# EUROPÄISCHE PATENTSCHRIFT

(11) **EP 0 335 827 B1**
(45) Veröffentlichungstag und Bekanntmachung des Hinweises auf die Patenterteilung: **28.07.1993**
(21) Anmeldenummer: 89730088.5
(22) Anmeldetag: 31.03.1989
(51) Int. Cl.: C07C 405/00, C07F 7/18, C07C 45/52

(54) **Neues Verfahren zur Herstellung von Bicyclo(3.3.0)octan-3-on-derivaten**
Process for the preparation of bicyclo(3.3.0)octan-3-one derivatives
Procédé de préparation de dérivés de bicyclo(3.3.0)octan-3-one

(30) Priorität: 31.03.1988 DE 3811577
(43) Veröffentlichungstag der Anmeldung: 04.10.1989
(73) Patentinhaber: SCHERING AKTIENGESELLSCHAFT, 13342 Berlin (DE)
(72) Erfinder: Skuballa, Werner, Dr., D-1000 Berlin 28 (DE)

(56) Entgegenhaltungen:
- FR-A- 2 534 917
- TETRAHEDRON LETTERS, Band 24, Nr. 37, 1983, Seiten 3943-3946, Pergamon Press Ltd, Oxford, GB; J.K. CHA et al.: "On stereochemistry of osmium tetroxide oxidation of allylic alcohol systems: empirical rule"

## Beschreibung

Die Erfindung betrifft ein neues Verfahren zur Herstellung von Bicyclo[3.3.0]-octanderivaten.

Bei der Synthese von (5E)-3-Oxa-6a-carba-prostaglandin-I₂-Derivaten der Formel II 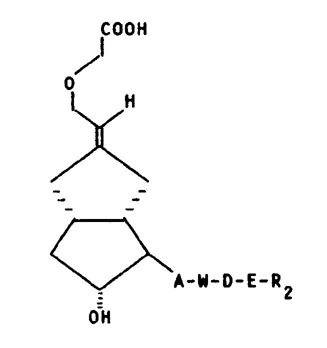
worin A, W, D, E und R₂ die in der allgemeinen Formel I definierten Bedeutungen haben, wird eine Stufe durchlaufen, die zu etwa 50% aus dem gewünschten Allylalkohol mit einer E-konfigurierten Doppelbindung 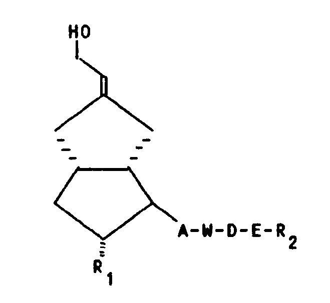
worin A, W, D, E, R₁ und R₂ die in der Formel I genannten Definitionen besitzen, und zu etwa 50% aus dem für die weitere Synthese unbrauchbaren Allylalkohol mit einer Z-konfigurierten Doppelbindung 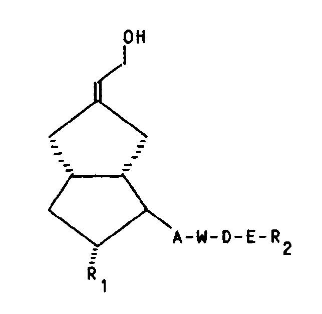
worin A, W, D, E, R₁ und R₂ ebenfalls die in der Formel I genannten Definitionen besitzen, besteht. In den Deutschen Offenlegungsschriften DE-OS 30 48 906, 33 06 123 und 33 06 125 werden derartige E-konfigurierte Allylalkohole zu pharmakologisch wirksamen (5E)-3-Oxa-6a-carba-prostaglandin-I₂-Derivaten, wie sie oben bereits genannt wurden, umgesetzt.

Es stellte sich also die Aufgabe, ein Verfahren zu finden, das diese wertvollen, jedoch falsch konfigurierten Allylalkohole zu Bicyclo[3.3.0]octan-3-on-Derivaten rückoxidiert und diese damit der vielstufigen Synthese der eingangs genannten (5E)-3-Oxa-6a-carba-prostaglandin-I₂-Derivate wieder zuführt. Es zeigte sich jedoch, daß sich die (Z)-Allylalkohole mit den üblichen Doppelbindungsspaltmethoden, wie der Perjodatspaltmethode, nur in sehr geringer Ausbeute in die gewünschten Bicyclo[3.3.0]octan-3-on-derivate der allgemeinen Formel I umsetzen lassen.

Es wurde nun überraschenderweise gefunden, daß man diese Allylalkohole, wenn man sie vorher in Ether oder Ester überführt hat, durch Hydroxylierung mit N-Methylmorpholin-N-oxid/Os0₄ und anschließende Behandlung mit Bleitetraacetat oder NaJO₄ zu den gewünschten Bicyclo[3.3.0]octan-3-on-derivaten in sehr guten Ausbeuten rückoxidieren kann.

Entsprechend Beispiel 1d) in DE-OS 33 06 123 erhält man aus den Bicyclo-[3.3.0]octan-3-onen nach Wittig-Horner-Reaktion und anschließender DIBAH- oder LiAlH₄-Reduktion 1:1-Gemische der E/Z-konfigurierten Allylalkohole, die chromatographisch in die gewünschten (E)-Allylalkohole und die (Z)-Allylalkohole getrennt werden, wobei die (Z)-Derivate dem erfindungsgemäßen Verfahren erneut zugeführt werden.

Die vorliegende Erfindung betrifft somit ein Verfahren zur Herstellung von Bicyclo[3.3.0]octan-3-on-derivaten der allgemeinen Formel I 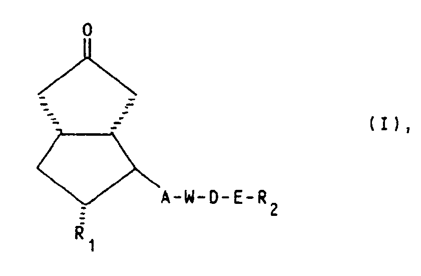
worin
- A: eine trans-CH=CH-Gruppe oder eine -C≡C-Gruppe,
- W: eine Hydroxymethylengruppe, in der die OH-Gruppe durch eine Trialkylsilylgruppe mit alkyl in der Bedeutung eines C₁-C₄-Alkyls, durch eine C₁-C₆-Alkanoylgruppe, durch Tetrahydropyranyl oder Tetrahydrofuranyl substituiert ist,
- D: eine 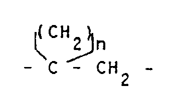 Gruppe mit n 1 bis 3, eine geradkettige Alkylengruppe mit 1-5 C-Atomen oder eine verzweigte Alkylengruppe mit 2-5 C-Atomen,
- E: eine -C≡C-Gruppe oder eine -CR₃=CR₄-Gruppe mit R₃ und R₄ jeweils in der Bedeutung eines Wasserstoffatoms, eines Chloratoms, eines Bromatoms oder einer C₁-C₄-Alkylgruppe,
- R₁: eine Hydroxygruppe, die wie die Hydroxygruppe in W substituiert ist,
- R₂: eine geradkettige oder verzweigte Alkylgruppe mit 1-7 C-Atomen,

das dadurch gekennzeichnet ist, daß man Bicyclo[3.3.0]octan-Derivate mit einer Z-konfigurierten Doppelbindung im 3-Ethylidenrest der allgemeinen Formel II, 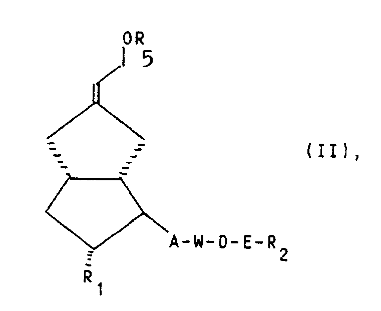
worin A, W, D, E, R₁ und R₂ die oben angegebenen Bedeutungen haben und R₅ eine C₁-C₆-Alkanoylgruppe, einen Benzoylrest, einen Cyclohexylcarbonylrest, einen Tetrahydropyranylrest, einen Tetrahydrofuranylrest oder eine Trialkylsilylgruppe mit alkyl in der Bedeutung eines C₁-C₄-Alkyls darstellt, mit N-Methylmorpholin-N-oxid in Gegenwart katalytischer Mengen Osmiumtetroxid hydroxyliert und mit Bleitetraacetat oder NaJO₄ spaltet.

Die in dem neuen Verfahren eingesetzten Ausgangsverbindungen werden in den Deutschen Offenlegungsschriften DE-OS 30 48 906, 33 06 123 und 33 06 125 beschrieben.

Zur Veretherung des Hydroxyls des Allylalkohols wird z.B. mit Dihydropyran in CH₂Cl₂ oder CHCl₃ unter Verwendung eines sauren Kondensationsmittels, wie beispielsweise p-Toluolsulfonsäure, umgesetzt. Das Dihydropyran wird im Überschuß eingesetzt, vorzugsweise in der 4- bis 10-fachen Menge des theoretischen Bedarfs. Die Umsetzung ist normalerweise bei 0-30°C nach 15-30 Minuten beendet. Die Acylate der Allylalkohole werden nach bekannten Methoden mit einem Carbonsäurederivat, wie z.B. Säurechlorid, Säureanhydrid u.a. in Gegenwart einer tertiären Base hergestellt.

N-Methylmorpholin-N-oxid wird in equimolaren Mengen und OsO₄ in katalytischen Mengen eingesetzt. Die Umsetzung der Verbindungen der Formel II mit diesen Reagenzien wird bei 0-80°, vorzugsweise 0-30°C, in 5 bis 48 Std., vorzugsweise 10-24 Std., in Keton/Wasser-Gemischen oder THF durchgeführt. Als Ketone kommen Aceton, Diethylketon, Methyl-isobutylketon u.s.w. infrage. Die Umsetzung mit Bleitetraacetat erfolgt in halogenierten Kohlenwasserstoffen wie CHCl₃, CH₂Cl₂, CCl₄, Perchlorethylen u.s.w. bei -70 bis + 50°C, vorzugsweise -30 bis +25°C, in 0,25 - 12 Std., vorzugsweise 0,5 - 2 Std. Bleitetraacetat wird dabei im Überschuß (1,1-1,2 molar) eingesetzt.

Die C₁-C₄-Alkylreste in der Trialkylsilylgruppe bzw. der Reste R₃ und R₄ können Methyl, Ethyl, n-Propyl, Isopropyl, n-Butyl, Isobutyl, sek.-Butyl oder tert.-Butyl bedeuten. Die C₁-C₆-Alkanoylgruppe R₅ kann Formyl, Acetyl, Propionyl, Butyryl, Pentanoyl oder Hexanoyl darstellen.

Für R₂ als geradkettige oder verzweigte Alkylgruppe mit 1-7 C-Atomen kommen Methyl, Ethyl, Propyl, Isopropyl, n-Butyl, Isobutyl, sek.-Butyl, n-Pentyl, sek.-Pentyl, Neopentyl, n-Hexyl, n-Heptyl in Frage. Bevorzugt sind Alkylgruppen mit 1-4 C-Atomen.
D als geradkettige Alkylengruppe mit 1-5 C-Atomen oder verzweigte Alkylengruppe mit 2-5 C-Atomen beeinhaltet folgende Reste:
methylen, ethylen, trimethylen, tetramethylen, pentamethylen,

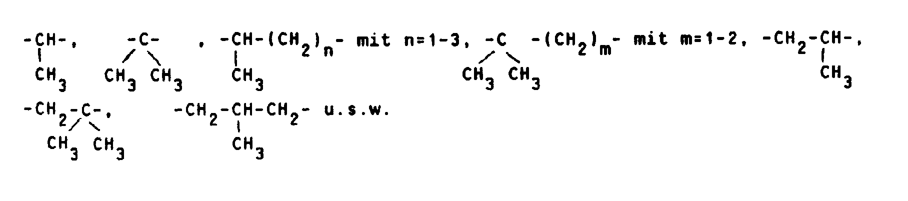

### I. Herstellungsvorschriften für das Ausgangsmaterial

### Vorschrift 1

### (1S,5S,6S,7R)-3-[(1Z)-(2-Acetoxyethyliden)]-7-tert,-butyldimethyl-silyloxy-6-[(3S,4S)-3-tert.-butyldimethylsilyloxy-4-methyl-1,6-nonadiinyl]-bicyclo-[3,3,0]octan

28g (1S,5S,6S,7R)-3-[(1Z)-(2-Hydroxyethyliden)]-7-tert.-butyl-dimethylsilyloxy-6-[(3S,4S)-3-tert.-butyldimethylsilyloxy-4-methyl-1,6-nonadiinyl]-bicyclo-[3.3.0]-octan werden in 100 ml Pyridin bei Raumtemperatur gelöst und nach Zugabe von 50 ml Acetanhydrid 16 Std. bei Raumtemperatur unter Argon gerührt. Das Reaktionsgemisch wird viermal jeweils mit 80 ml Toluol versetzt und am Rotationsverdampfer eingeengt. Danach wird der Rückstand erst im Wasserstrahlpumpenvakuum und danach im Ölpumpenvakuum getrocknet. Es entstehen 30 g eines farblosen Öls, das dünnschichtchromatographisch [Hexan/Ether (6:4), Anfärben mit H₂SO₄/Ethanol und Erhitzen] keine nennenswerten Nebenprodukte zeigt.

### Vorschrift 2

### (1S,5S,6R,7R)-3-(2-Acetoxy-1-hydroxyethyl)-7-tert.-butyldimethyl-silyloxy-6- [(3S,4S)-tert.-butyldimethylsilyloxy-4-methyl-1,6-nonadiinyl]-bicyclo[3.3.0]-octan-3-ol

30g der nach Vorschrift 1 hergestellten Verbindung werden in 860 ml Aceton/ 125 ml Wasser gelöst. Danach werden bei 0°C und Argon-Atmosphäre unter Rühren 6,53g N-Methyl-morpholin-N-oxid zugefügt. Anschließend wird über einen Zeitraum von 1 Std. eine Lösung aus 0,5 g Osmiumtetroxid in 250 ml tert.-Butanol zugetropft, 1 Std. bei Eisbadtemperatur und 16 Std. bei Raumtemperatur gerührt. Nach Einengung am Rotationsverdampfer wird der Rückstand auf 300 ml Eiswasser gegossen, viermal mit je 300 ml Essigester extrahiert, die organische Phase mit 200 ml Sole gewaschen und nach Trocknung über Na₂SO₄ eingedampft. Durch Chromatographie des Rückstandes an Kieselgel mit Hexan/Essigester (1:1) erhält man 24,1 g (75% der Theorie) eines farblosen Öls.

### II. Beispiele

### Beispiel 1

### (1R,5S,6S,7R)-7-tert.-Butyldimethylsilyloxy-6-[(3S,4S)-4-methyl-3-tert.-butyl-silyloxy-1,6-nona-diinyl]-bicyclo[3.3.0]octan-3-on

24,7g des nach Vorschrift 2 erhaltenen Diols werden in 800ml Methylenchlorid gelöst. Danach wird die Lösung auf -20°C gekühlt. Bei dieser Temperatur werden 18,6g Bleitetraacetat zugefügt und 1 Std. bei -20°C gerührt. Nach Einengen des Reaktionsgemisches am Rotationsverdampfer wird der Rückstand in Wasser/Ether aufgenommen, die wässrige Phase dreimal mit je 200 ml Ether extrahiert, die Etherphase einmal mit 200 ml Wasser, zweimal mit je 200 ml 4%iger NaHCO₃-Lösung und zweimal mit Sole gewaschen, über Natriumsulfat getrocknet und schließlich am Rotationsverdampfer eingeengt. Der Rückstand wird an Kieselgel mit Hexan/Essigester (4:1) chromatographiert.
Ausbeute 17,4 g (85% der Theorie eines farblosen Öls).

## Patentansprüche

1. Verfahren zur Herstellung von Bicyclo[3.3.0]octan-3-on-derivaten der allgemeinen Formel I, 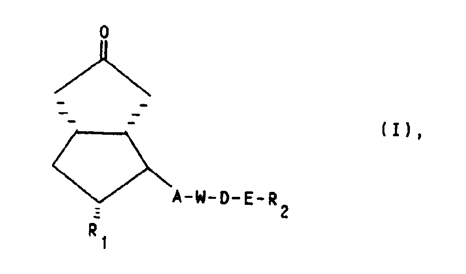 worin
A eine trans-CH=CH-Gruppe oder eine -C≡C-Gruppe,
W eine Hydroxymethylengruppe, in der die OH-Gruppe durch eine Trialkylsilylgruppe mit alkyl in der Bedeutung eines C₁-C₄-Alkyls, durch eine C₁-C₆-Alkanoylgruppe, durch Tetrahydropyranyl oder Tetrahydrofuranyl substituiert ist,
D eine 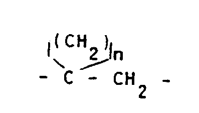 Gruppe mit n 1 bis 3, eine geradkettige Alkylengruppe mit 1-5 C-Atomen oder eine verzweigte Alkylengruppe mit 2-5 C-Atomen,
E eine -C≡C-Gruppe oder eine -CR₃=CR₄-Gruppe mit R₃ und R₄ jeweils in der Bedeutung eines Wasserstoffatoms, eines Chloratoms, eines Bromatoms oder einer C₁-C₄-Alkylgruppe,
R₁ eine Hydroxygruppe, die wie die Hydroxygruppe in W substituiert ist,
R₂ eine geradkettige oder verzweigte Alkylgruppe mit 1-7 C-Atomen,
dadurch gekennzeichnet, daß man Bicyclo[3.3.0]octan-Derivate mit einer Z-konfigurierten Doppelbindung im 3-Ethylidenrest der allgemeinen Formel II, 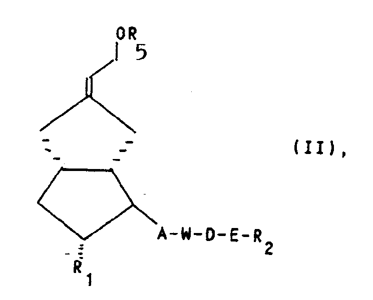 worin A, W, D, E, R₁ und R₂ die oben angegebenen Bedeutungen haben und R₅ eine C₁-C₆-Alkanoylgruppe, einen Benzoylrest, einen Cyclohexylcarbonylrest, einen Tetrahydropyranylrest, einen Tetrahydrofuranylrest oder eine Trialkylsilylgruppe mit alkyl in der Bedeutung eines C₁-C₄-Alkyls darstellt, mit H-Methylmorpholin-N-oxid in Gegenwart katalytischer Mengen Osmiumtetroxid hydroxyliert und mit Bleitetraacetat oder HaJO₄ spaltet.

## Claims

1. Process for the preparation of bicyclo[3.3.0]octan-3-one derivatives of the general formula I 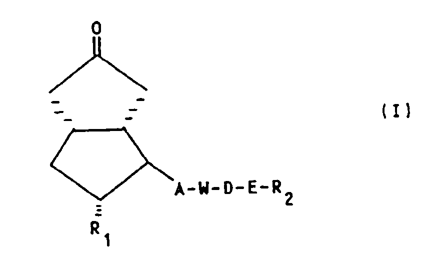 wherein
A is a trans-CH=CH- group or a -C≡C- group,
W is a hydroxymethylene group in which the OH group is substituted by a trialkylsilyl group in which alkyl is C₁-C₄alkyl, by a C₁-C₆alkanoyl group, by tetrahydropyranyl or by tetrahydrofuranyl,
D is a 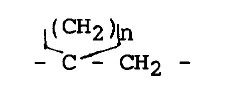 group in which n is from 1 to 3, a straight-chain alkylene group having from 1 to 5 carbon atoms or a branched alkylene group having from 2 to 5 carbon atoms,
E is a -C≡C- group or a -CR₃=CR₄- group in which R₃ and R₄ are each a hydrogen atom, a chlorine atom, a bromine atom or a C₁-C₄alkyl group,
R₁ is a hydroxy group that is substituted in the same way as the hydroxy group in W,
R₂ is a straight-chain or branched alkyl group having from 1 to 7 carbon atoms,
characterised in that bicyclo[3.3.0]octane derivatives having a Z-configured double bond in the 3-ethylidene radical of the general formula II 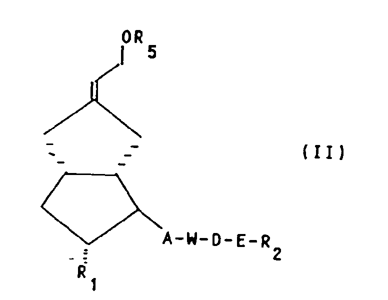 wherein A, W, D, E, R₁ and R₂ are as defined above and R₅ is a C₁-C₆alkanoyl group, a benzoyl radical, a cyclohexylcarbonyl radical, a tetrahydropyranyl radical, a tetrahydrofuranyl radical or a trialkylsilyl group in which alkyl is C₁-C₄alkyl, is hydroxylated with N-methylmorpholine-N-oxide in the presence of catalytic amounts of osmium tetroxide and cleaved with lead tetraacetate or NaIO₄.

## Revendications

1. Procédé de préparation de bicyclo[3.3.0]octane-3-ones de formule générale I 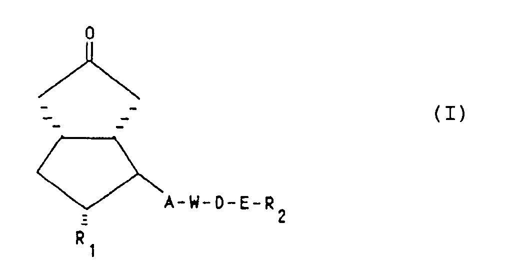 (dans laquelle
A représente un groupe trans-CH=CH- ou -C≡C-,
W un groupe hydroxyméthylène dont le groupe OH à comme substituant un trialkylsilyle à alkyle en C₁-C₄ ou bien un alcanoyle en C₁-C₆ ou un groupe tétrahydropyranyle ou tétrahydrofuranyle,
D un groupe 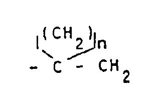 - ,n étant un nombre de 1 à 3, un alkylène à chaîne linéaire en C₁-C₅ ou un alkylène ramifié en C₂-C₅,
E groupe -C≡C- ou -CR₃=CR₄-, R₃ et R₄ désignant chacun un atome d'hydrogène, de chlore ou de brome ou un alkyle en C₁-C₄,
R₁ un hydroxyle substitué de la même manière que celui du groupe W ci-dessus et
R₂ un alkyle à chaîne linéaire ou ramifiée en C₁-C₇),
procédé caractérisé en ce que l'on hydroxyle avec du N-oxyde de N-méthylmorpholine en présence de proportion catalytique de tétroxyde d'osmium des bicyclo[3.3.0.]octanes à double liaison de configuration (Z) du radical 3-éthylidène, de Formule générale II 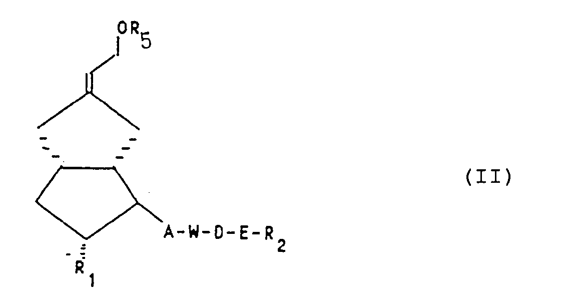 (dont les divers symboles ont les significations ci-dessus indiquées et R₅ désigne un alcanoyle en C₁-C₆ ou bien un radical benzoyle, cyclohexyl-carbonyle, tetrapyranyle, tetrahydrofuranyle ou trialkylsilyle à alkyle en C₁-C₄), puis on effectue la scission avec du tétra-acétate de plomb ou du periodate de sodium.
